Europäisches Patentamt

European Patent Office

Office européen des brevets

(19)

(11)  EP 1 421 958 A1

## (12) EUROPEAN PATENT APPLICATION
published in accordance with Art. 158(3) EPC

(43) Date of publication:
    26.05.2004  Bulletin 2004/22

(21) Application number: 02753211.8

(22) Date of filing: 31.07.2002

(51) Int Cl.⁷: **A61M 1/34**, A61M 1/14

(86) International application number:
    PCT/JP2002/007777

(87) International publication number:
    WO 2003/011367 (13.02.2003 Gazette 2003/07)

(84) Designated Contracting States:
    AT BE BG CH CY CZ DE DK EE ES FI FR GB GR
    IE IT LI LU MC NL PT SE SK TR
    Designated Extension States:
    AL LT LV MK RO SI

(30) Priority: 01.08.2001 JP 2001234141

(71) Applicant: JMS Co., Ltd.
    Hiroshima-shi, Hiroshima 730-0812 (JP)

(72) Inventor: IKEDA, Atushi,
    JMS CO., LTD., Chiyoda Factory
    Chiyodacho, Yamagata-gun, Hiroshima 731 (JP)

(74) Representative: Illingworth-Law, William et al
    D Young & Co
    21 New Fetter Lane
    GB-London EC4A 1DA (GB)

## (54) BLOOD PURIFICATION APPARATUS FOR ELEVATING PURIFICATION EFFICIENCY

(57)     The present invention relates to a blood purification apparatus characterized by having a blood osmotic pressure changing means that changes periodically the difference of osmotic pressure between the patient's blood osmotic pressure and the intracellular osmotic pressure of the patient. By using this apparatus, the following effects can be obtained.

(1) The blood purification can be carried out accurately by a simple operation.
(2) The blood purification can be carried out safely and physiologically for a living body.
(3) The dialysis efficiency can be improved (elevated). Thus, as a result, the extension of the blood purification time can be avoided and the removal amount of medium-molecular-waste products is increased.

Figure 1

EP 1 421 958 A1

## Description

### Technical Field

The present invention relates to a blood purification apparatus that can elevate blood purification efficiency, more concretely to a blood purification apparatus that can control blood purification by confirming the patient's blood condition, and that can elevate purification efficiency such as water removal or removal of waste products, solutes and the like.

### Background Art

For treating patients with impaired kidney function, blood purification such as hemodialysis or peritoneal dialysis and the like have been carried out conventionally. Hemodialysis is a treatment to purify blood by removing extra water or waste products and toxic agents in the blood, via semipermeable membrane in form of hollow fiber. Thus, as for an apparatus that carries out hemodialysis, it is important to maintain adequately the patient's blood condition (blood volume circulating in the body) to carry out safe and effective hemodialysis. In case the target dialysis condition is an excessive setting for the patient, the blood circulating volume will decrease excessively and it may cause reduction of blood pressure or shock. On the contrary, if the dialysis condition is too gentle, there is a problem of hypertension or heart failure caused by insufficient water removal. Furthermore, naturally, it will be hard to improve symptoms of kidney failure according to insufficient dialysis.

Therefore, a hemodialysis apparatus performing water removal by monitoring patient's blood condition have been proposed. For example, an apparatus that controls dialysis treatment by carrying out hemodialysis by measuring Hematocrit level, and that gears and controls blood pump, water removal pump and liquid supply pump, according to the blood circulating volume calculated from the Hematocrit level can be exemplified. The apparatus mentioned above has a simple construction so that anyone can use, but on the contrary the adjustment of the dialysis time is difficult (extension of dialysis time is fatal), therefore, there was a problem that the control of the former part of the dialysis cannot be performed and that it is effective only to the reduction of blood pressure of the latter part.

In Japanese Laid-Open Patent Application No. 9-149935, a hemodialysis apparatus that controls the dialysis condition by monitoring the blood condition is disclosed. Moreover, in Japanese Laid-Open Patent Publication No. 6-83723, a controlling apparatus that estimates the body fluid condition with the Hematocrit level, and controls the blood pump or ultra pressure according to said condition. However, as for the apparatus described above, there were problems that there were no safety mechanism to suppress out-of-control of the feedback control, or that the operator had to operate dialysis condition or the apparatus each time the blood condition departs from the defined condition.

Furthermore, the present inventors also have proposed in the past, a blood purification apparatus that can carry out hemodialysis treatment by monitoring the patient's blood condition. For example, the blood purification apparatus disclosed in Japanese Patent Application No. 10-101324 (Japanese Laid-Open Patent Application No. 11-221275) can be exemplified. However, as this apparatus gains the water removal volume in the former part of the dialysis, it has a merit of being able to control the blood circulating volume during dialysis within the predetermined range which can solve the problem of extension of dialysis time. But, on the contrary, it had demerits that it was necessary to define an alarm zone to control, that it was necessary to define controlling parameters in detail.

There were not only problems concerning hemodialysis apparatus as described above, but also essential problems more important concerning the hemodialysis itself. These are problems of hemodialysis concerning dialysis efficiency such as solute removal, water removal and the like, which are disadvantages unavoidable for hemodialysis membrane. In conventional hemodialysis, the removal of low-molecular-weight waste products was satisfactory, but as the removal efficiency of medium-molecular-weight was not good, it was provided to use a hollow fiber dialysis membrane with a larger diameter of the hole, to increase the removal efficiency of medium-molecular-weight solutes. However, by using dialysis membrane with a large diameter of the hole, the removal of medium-molecular-weight was improved but on the other hand, there were defects that albumin or other protein necessary for a living body were removed together.

### Disclosure of the Invention

The object of the present invention is to solve the above-mentioned problems. That is, it is to provide a blood purification apparatus that can carry out blood purification accurately and easily, and that can carry out blood purification safely and physiologically for a living body, and moreover that can improve (elevate) the dialysis efficiency.

More concretely, first, it is to provide a blood purification apparatus that does not need to perform complicate setting or controlling operation, and that can attain the target blood purification treatment.

Second, it is to provide an apparatus that can carry out physiological (gentle) dialysis to each patient, in a safe way so that the patient would not be in a dangerous condition by the excessive purification treatment, and that the patient can receive the purification comfortably□.

Third, it is to provide a blood purification apparatus that have elevated (improved) the dialysis efficiency shown by removal of solute such as waste products and the like or water removal.

Forth, it is to provide a blood purification apparatus that can improve the removal efficiency of medium-molecular-weight waste products, while maintaining protein necessary for a living body.

To achieve said object, the present inventors have formed artificially an osmotic gradient (in other words, a difference of osmotic pressure) between the osmotic pressure of patient's blood and the intracellular osmotic pressure of the patient during blood purification, specially an artificial repeating condition of increasing and decreasing (rise and fall) to the blood osmotic pressure in the blood circuit of the blood purification apparatus, so that the high pressure condition and the low pressure condition of osmotic pressure in patient's blood vessel is alternated, to dispose a difference with the intracellular osmotic pressure, and to improve efficiency of water removal or solute removal, that was made to be a basic technical philosophy. Thus, the present invention was completed according to this technical philosophy.

In other words, the present invention provides a blood purification apparatus having a blood osmotic pressure changing means that forms artificially an osmotic gradient between the osmotic pressure of patient's blood and the intracellular osmotic pressure of the patient during blood purification, especially a blood osmotic pressure changing means that forms by changing periodically said osmotic gradient especially in at least one part of the process of the blood purification, especially in the latter process of the blood purification process.

As for a means that forms the osmotic gradient between the osmotic pressure of patient's blood and the intracellular osmotic pressure of the patient mentioned above, the change of purification condition of the blood purification process and the use of osmotic substance, (material forming the osmotic gradient) can be exemplified.

As a technical means that changes the osmotic gradient artificially and periodically, a means that changes the purification condition of the blood purification process cyclically can be exemplified, and for example there is a means by performing the change of water removal speed, including the stop of water removal.

By changing water removal speed, for example by repeating increasing and decreasing of the water removal speed cyclically, or repeating the high water removal speed and stop of water removal cyclically, the difference between the blood osmotic pressure and the intracellular osmotic pressure is formed periodically and that can elevate the water removal efficiency or solute removal efficiency. Furthermore, the ability of removing specific solute (especially medium-molecular-weight solutes and the like which are difficult to move) can be improved.

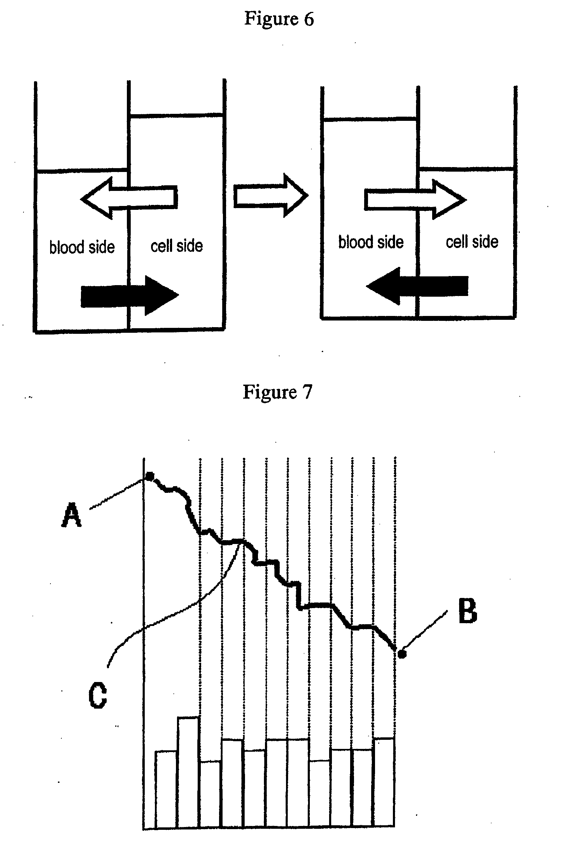

For the reason that the efficacy of the blood purification as mentioned above can be reached, it is estimated that it is due to a phenomenon as follows, and is explained according to Figure 6.

When the water removal speed is high, the blood which the concentration condition of blood is high will recycle in the body. Therefore, the osmotic gradient between blood and cells will become larger, and the material transfer between cells and blood will become larger. That is, when the blood side is more concentrated than the cells, water will move from cells to blood side. At the same time, the components of blood side will transfer to cells. Due to the difference of the transfer speed of the solute and the solvent that transfer, the concentration of plasma components will vary temporarily. Therefore, as it is shown in Figure 6 (a), water to correct the difference of osmotic pressure (white arrow) will flow in from cells, and the solute components (black arrow) will transfer into cells, the concentration of small molecule of plasma components will decrease temporarily. Then, the osmotic gradient of cells and the plasma will decrease□gradually and stabilizes (it is believed that it stabilizes where the osmotic pressure is higher than usual). When the water removal speed is stopped, the plasma components will no more have the concentration action according to water removal, and simply will be only the action according to material transfer and diffusion efficacy according to the difference between osmotic pressure of dialysate and plasma, and the osmotic pressure of plasma will show a lower level than when performing normal water removal. Moreover, when the dialysis is carried out with a water removal speed faster than usual, at the previous stage of the stop of water removal it is believed that the intracellular osmotic pressure will be a higher level than usual. Thus, as it is shown in Figure 6 (b), on the contrary, water will flow in from the blood side into cells, and the solute components will move to blood vessel side. Furthermore, when cells of said condition and the blood of lower osmotic pressure than usual will meet, a higher efficacy of material transfer than usual (not only transfer of water or small-molecule but transfer of medium or large molecular weight) can be expected. When a high water removal speed and stop of water removal are repeated cyclically, according to the difference between the cycle and the transfer speed of each solute, it is expected the removal ability of specific material is improved (by combining cycle, water removal speed, dialyzer, dialysate and the like, the ability of component removal from cells will vary).

As for another technical means that changes periodically the osmotic pressure of patient's blood and the intracellular osmotic pressure of the patient, there is a means that supplies osmotic pressure changing material into a blood circuit or a circuit supplying dialysate, to form artificially the repeating condition of increasing and decreasing of the patient's blood osmotic pressure.

To change the blood osmotic pressure circulating in the blood circuit by using an osmotic pressure changing material, there is a means, for example, that provides a liquid supply means in the blood circuit and connect the osmotic pressure changing means to it, and a means that changes the osmotic pressure of the dialysate circulating in the dialysate circuit.

The latter means is an injector of fluid comprising osmotic pressure changing material that can changes the content volume of the osmotic pressure changing material of the dialysate circulating in the dialysate circuit, for example an injector of fluid comprising sodium (hereinafter also referred as sodium injector) which is an apparatus injecting said dialysate (via endotoxin filter) into the blood circuit. Comparing to the former one, the latter can be realized in an affordable price and by using with a method that supplies dialysate by an on-line mechanism hereinafter described, it can change the blood osmotic pressure rapidly and in a wide range.

In case of injecting fluid comprising sodium as an osmotic pressure changing material into the dialysate, it is preferable that the concentration is high content, for example 145-150 mEq/l to increase blood osmotic pressure, and that the concentration is low content, for example 140-143 mEq/l to lower the blood osmotic pressure.

As for said blood purification method, method of hemodialysis, hemofiltration (HF) and hemodiafiltration (HDF) can be exemplified, and among these, HF or HDF supplying dialysate by on-line is preferable, and especially, HDF is preferable as it uses UFR controller to make the fluid volume flowing in the dialyzer same as the fluid volume flowing out, and can change the blood osmotic pressure rapidly, sharply and in a wide range.

As for material to be used to perform operation of changing periodically the blood osmotic pressure to be high and low artificially as mentioned above, it has no specific limitation as long as it is a material that can achieve said object, and osmotic pressure changing material such as electrolytes selected from sodium, calcium and magnesium, alkalizers selected from lactic acid and bicarbonic acid, and glucose, or a combination thereof and the like can be exemplified. Among these, osmotic pressure changing material comprising sodium ion is preferable from the point of view of safety and effectiveness.

The present invention will be explained concretely in the following.

1. Blood purification using blood osmotic pressure changing means.

The former part of blood purification

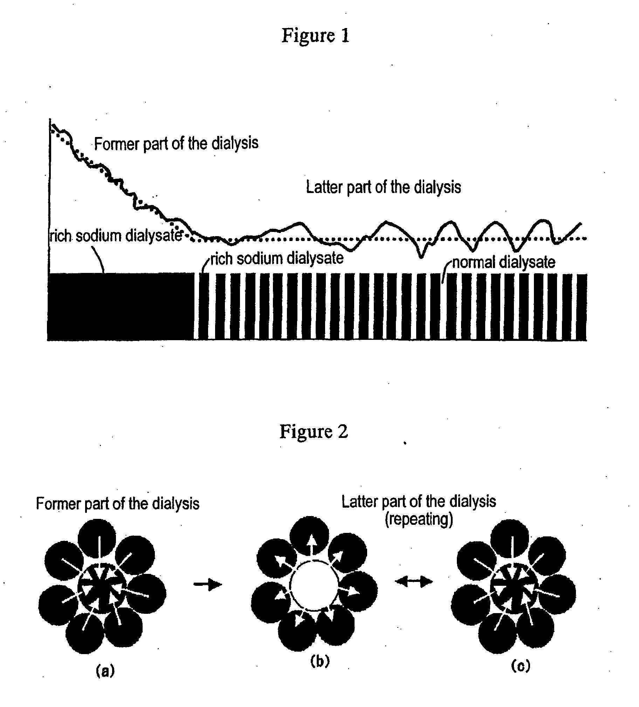

As it is shown in Figure 1 and Figure 2(a), in the former part of blood purification, it is preferable to form a high difference of osmotic pressure between blood osmotic pressure and intracellular osmotic pressure of the patient, for example by injecting continuously high electrolytic solution from dialysis circuit into blood circuit by on-line HDF to keep high the blood osmotic pressure in blood vessel 3. In other words, in the former part of the blood purification process of the present invention, it is preferable that the apparatus is constituted so that it can control to carry out the purification process so that the blood osmotic pressure is continuously in an increasing state, using a blood osmotic pressure changing means as described above, to achieve the object of the present invention. For example, as it is shown in Figure 1, in the former part of the blood purification process, for example the dialysis efficiency can be further elevated by performing a fluid supply in a high Na condition continuously, and by increasing plasma osmotic pressure to increase PRR to elevate UFR (water removal speed).

Furthermore, as for the blood purification operation with the use of blood osmotic pressure changing means as mentioned above, it is preferable that it controls by controlling blood volume (BV) with the use of a blood indication level, as described hereinafter.

As a result that the blood purification operation as described above is carried out, and for example when the blood volume attains the standard blood volume, the target BV% level or time that were defined, the latter part of the blood purification is started.

The latter part of the blood purification

In case the blood purification is carried out with the use of blood purification apparatus of the present invention, using the difference of osmotic pressure between patient's blood osmotic pressure and intracellular osmotic pressure of the patient, it is preferable that the means forming artificially the repeating condition of increasing and decreasing of blood osmotic pressure as described above is performed in the latter part of the blood purification process.

For example, from the time when the latter part of the blood purification is started, it is controlled so that the blood osmotic pressure is alternatively repeating a high condition and a low condition, by performing alternatively the injection of high electrolyte fluid and the injection of normal dialysate, from dialysate supplying circuit into blood circuit in on-line HDF. It is preferable that the number of times of said repetition is at least more than 2 times (low-high-low), but it is complicated and causes problems if the number of times is too many. Moreover, as the dialysis time is almost determined, the number of times of repetition is also associated with the cycle of increasing and decreasing of blood osmotic pressure (low-low, or high-high). The efficiency will hardly be shown if the cycle is too long or too short, thus the preferable cycle is defined accordingly.

In the following, an example of controlling method that controls so that the blood osmotic pressure repeats alternatively a high condition and a low condition in the latter part of the blood purification will be described.

The blood osmotic pressure (plasma osmotic pressure) in blood vessel 3 is lowered by monitoring the blood indication level and by performing normal liquid supply. Therefore, the osmotic pressure in cell 2 becomes relatively high compared to blood osmotic pressure, and the transfer of water 1 from blood vessel 3 into cell 2 is started. Therefore, the osmotic pressure of cell 3 is lowered, and the water removal is stopped using the blood indication level defined beforehand. In figure 2 (b), the view showing a frame format of the transfer of water 1 from blood vessel 3 into cell 2 is shown. Then, high electrolyte fluid is injected again from the dialysate circuit into the blood circuit by on-line HDF mechanism. As a result, the blood osmotic pressure in blood vessel 3 increases, and the water will flow into blood vessel 3 from inside of cell 2 wherein the osmotic pressure became relatively low. According to this, as it is shown in Figure 2 (c), the solute in cell 2 will flow into blood vessel 3. Thus, by changing artificially the blood osmotic pressure to be high and low periodically, the dialysis efficiency will elevate as the efficiency of water removal or solute removal will elevate with the difference between osmotic pressure in the blood vessel and in the cells. As a result, medium-molecular-weight solute that are difficult to transfer will be easier to be removed. Furthermore, in the latter part of the blood purification, it will also be possible to carry out dialysis by maintaining PRR, and it will be possible to define UFR higher than conventional and as a result to prevent the extension of the dialysis time.

Next, the reason why it is preferable to adapt a means that forms artificially the repeating condition of increasing and decreasing of the blood osmotic pressure in the latter part of the blood purification will be explained.

In the former part of the blood purification, the water volume inside the blood vessel and inside the cells is high, and there is no space neither in the blood vessel nor in the cells to exchange water. When said space is small, the efficiency of flowing in and out is not good. On the contrary, it is preferable to form a repeating condition of said osmotic pressure, at the time after the water removal of the former part of the blood purification process is performed, water inside the blood vessel and inside the cells is removed, and a space so that water can flow in is made. Thus, it is easy to make blood hyperosmotic and as the water volume flowing in and out, occurred by the difference of osmotic pressure is large, it is possible to perform efficient water exchange.

In the meantime, as for the aspect of the description of the latter part of the blood purification process of the present invention described above, it is a relative aspect wherein the purification process is divided in two at an optional point, and the purification process part before said point is called the former part of the purification process, and the purification process part after said point is called the latter part of the purification process. However, it is preferable that the purification operation is started as described above and the purification is performed by controlling BV, and for example that the part after the blood volume attains the standard blood volume or the target BV% level which were defined is called the latter part of the purification process.

In the hemodialysis treatment with the use of said blood osmotic pressure changing means described above, when forming a difference of blood osmotic pressure as described above, it would be possible to perform blood purification treatment in a condition adequate to each patient or in a physiological condition, by changing blood osmotic pressure or performing water removal by confirming the blood volume (BV level) or the blood volume change (hereinafter also referred as blood indication level) described hereinafter. Thus, as for the blood purification apparatus of the present invention, it is preferable that the apparatus has a means enable to measure or calculate said blood indication level and a controlling means that controls the increasing and decreasing of the blood osmotic pressure by said means, with a blood osmotic pressure changing means.

As for the blood purification apparatus of the present invention, it will be possible not only to carry out blood purification under condition suitable to patient, but also to carry out more efficient blood purification by controlling the repeating condition of increasing and decreasing of the blood osmotic pressure according to the blood volume described above or each blood indication level described above.

As for a controlling method of a blood purification method with the use of blood indication level such as described above, examples include: a blood purification method defining a target control line constituted with a blood indication level described hereinafter, and controlling blood purification using said target control line; especially a blood purification method wherein the blood purification is carried out according to said target control line in the former part of the dialysis and the latter part of the blood purification is started at the point when the blood volume attains the standard blood volume, target BV% level or time that were defined. As a method for controlling blood purification using a target line such as described before, controlling methods disclosed in Japanese Patent Application No. 2002-19447 or PCT/JP02/06744, filed previously by the present applicants can be exemplified.

Moreover, the blood purification apparatus of the present invention record into a medium as a software, purification condition and blood indication level and the like such as described above, and the blood purification can also be carried out using said medium. Thus, the present invention has also as object of invention, such software and record medium itself.

In the following, blood indication levels such as BV level used for control in the blood purification apparatus of the present invention will be explained.

(1) BV level

BV level is the abbreviation of Blood Volume level and is the index of circulating blood volume which is the indication level to check the condition of the circulating blood volume of each patient.

(2) Standard blood volume

In a human body, when kidney is functioning normally, adjustment of water is performed to maintain the osmotic pressure of intracellular fluid. As a result, the blood volume will be maintained in a certain range. However, when the kidney function is impaired and a trouble occurs in the water adjustment mechanism of the body, water will be pooled in the blood vessel and cells. Moreover, when the solute removal ability would not function, the osmotic pressure in the blood or cells will increase, and the mechanism to stock more water in blood vessels and cells will operate to resist it. However, as neither blood vessel nor cells cannot stock water infinitely, even if the osmotic pressure in the cells or plasma increases, there is a limit to lower the osmotic pressure by pooling water. From these points, it is estimated that the osmotic pressure in the cells or plasma of patients before receiving blood purification, is a higher value compared to a person with normal kidney function. Then, when the blood purification such as hemodialysis is started, the water removal from the blood is performed with the solute removal, and the osmotic pressure will decrease. However, as the water removal is performed from the blood, the plasma osmotic pressure will not decrease drastically. During dialysis, despite there is possibility that the plasma osmotic pressure will be lower than the intracellular osmotic pressure, the reason why PRR from cells occur is that the turgor pressure in the cells is high, and water will flow in the space inside the blood vessel formed after the water removal. In the latter part of the dialysis, when the turgor pressure in the cells decrease, and a space is formed also in the cells, there will be possibility that water transfers from blood into cells, and reduction of blood pressure will occur easily according to the difference of the osmotic pressure and the turgor pressure. Therefore, it is preferable that as for the blood volume to maintain during dialysis, the blood volume maintained normally is believed to be the standard blood volume, and that the water removal is performed to maintain that condition. Then, in the primary part of the dialysis, it is believed that the blood volume is increased to be more than the standard blood volume, and that it is preferable to perform the water removal to decrease the patient's blood volume rapidly to the standard blood volume in the former part of the dialysis, and after the blood volume is lowered substantively to the standard blood volume, to perform the water removal by maintaining said standard blood volume. The standard blood volume is a blood volume which the patient would have if healthy, by considering factors that might influence the human blood volume, for example the patient's age, sex, body height and the like, defined beforehand by doctors and the like.

2. Primary blood volume ($BV_0$)

(1) The meaning of measuring the primary blood volume ($BV_0$)

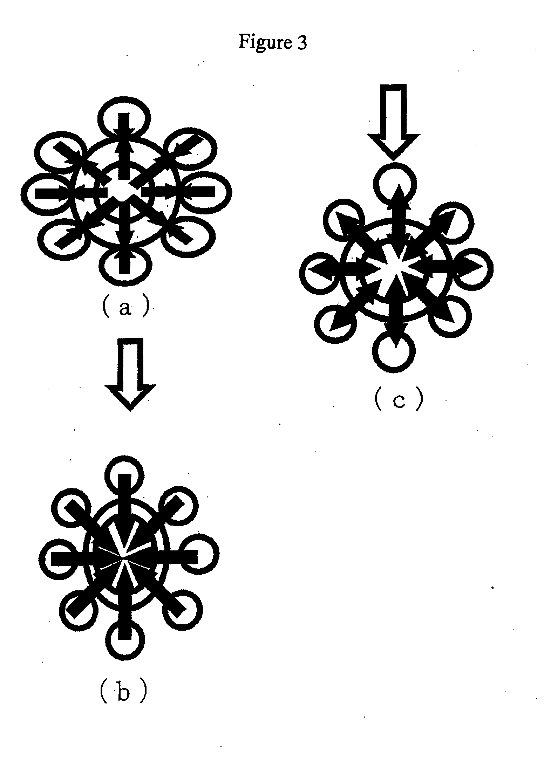

The meaning of measuring the primary blood volume is explained according to Figure 3 by taking dialysis as an example.

As it is shown in Figure 3 (a), in the body of dialysis patient before starting circulation outside the body, both the cells and blood vessels are expanded up to near the limit. When the circulation outside the body is started, the volume of the blood vessel part is increased, and a space where water can flow in is formed inside the blood vessel. Therefore, as it is shown in Figure 3 (b), water will flow into the blood vessel from the cells, and just after the blood circulation outside the body is started, the blood volume becomes unstable. When the space inside the blood vessel is filled up with water flowed in from cells, and the sum of the turgor pressure inside the blood vessel and inside the cells and the osmotic pressure will be balanced out, the inflow of water will be stopped as it is shown in Figure 3 (c). Therefore, as for the measuring time of Hematocrit level being the calculating basis of the patient's primary blood volume, it is preferable to be a little later than the initiation of the circulation outside the body, to obtain an accurate blood volume. Moreover, just after the circulation outside the body is started, it is necessary to wait until the blood volume stabilizes and not to perform water removal.

(2) Calculating primary blood volume

a. First calculating method (a method of obtaining the primary blood volume by the change of the water removal speed)

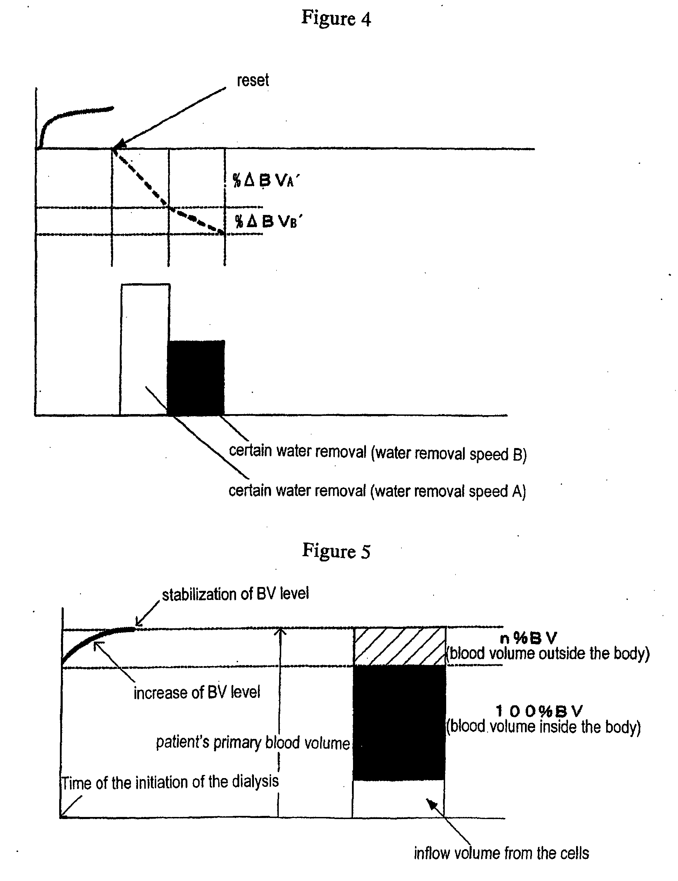

The calculating method is explained according Figure 4, by taking dialysis as an example.

As it is shown in Figure 4, at the time of the initiation of the dialysis only circulation outside the body is performed, and circulation outside the body is continued until BV level stabilizes, and when the BV level becomes stable, dialysis accompanied by water removal is initiated, and at the same time as said dialysis is initiated, the water removal is performed with a certain time (within the time PRR does not change), and with a certain water removal volume (water removal volume 1), and thus $\Delta BV_1\%$, which is the volume change of said BV level is calculated. Then, the water removal is performed for the same time as said certain time with a different water removal volume (water removal volume 2), and thus, $\Delta BV_2\%$ which is the volume change of said BV level, is calculated. Thus, $BV_0$ can be calculated by using said $\Delta BV_1\%$, said $\Delta BV_2\%$, water removal volume 1 and water removal volume 2.

Then, the following relational formula is obtained among the change of the blood volume, the volume flowing in from the cells (PRR), the water removal volume, the ratio of blood volume change ($\Delta BV\%$) and the primary blood volume.

$$BV_0 = \text{(water removal speed A - water removal speed B)}/(-$$

$$\Delta BV_1\% + \Delta BV_2\%) \times \Delta T$$

Said formula can be calculated as follows. The calculating method is explained according to Figure 4.

$$\Delta BV/\Delta T = PRR - UFR$$

$$-\Delta BV_1/\Delta T + \Delta BV_2/\Delta T = \text{water removal speed A - water removal}$$

$$\text{speed B}$$

$$BV_1 = BV_0 \ \square \ \%\Delta BV_1$$

$$\Delta BV_2 = BV_0 \ \square \ \%\Delta BV_2$$

$$BV_0 /\Delta T (-\%\Delta BV_1 + \%\Delta BV_2) = \text{water removal speed A - water}$$

$$\text{removal speed B}$$

$$BV_0/\Delta T = \text{(water removal speed A - water removal speed B)} /$$

$$(-\%\Delta BV_1 + \%\Delta BV_2)$$

b. Second calculating method (a method of obtaining the primary blood volume without performing water removal)

The calculating method is explained according Figure 5, by taking dialysis as an example.

Before starting the circulation outside the body, the water volume that can be pooled inside blood vessel has attained the maximum level for dialysis patient, and the blood volume before the dialysis is thought to be a fixed level per patient. As the circulation outside the body is initiated, the (blood) circulating volume will increase by the volume in the circulation circuit outside and a space to accept water corresponding to the increased blood circulating volume in the blood inside the body (blood vessel) is generated. This is why the blood indication level associated with the Hematocrit level or the blood volume is increased in the primary part of the dialysis. At said time of initiation of the circulation outside the body, when the circulation outside the body is continued until the BV level stabilizes without

**EP 1 421 958 A1**

performing water removal, it is believed that water corresponding to the increased blood circulating volume mentioned above, is transferred from the cells into the blood vessel (provided the turgor pressure in the cells is sufficiently high, water run over the cells and is accumulated up to the cell stroma). At that time, when the circulation outside the body is continued without water removal, the blood volume will increase by the same volume of water that has been transferred from the cells to the blood vessel. Said increased blood volume is approximately the volume inside the circuit (circulation outside the body), and as the volume inside the circuit is possible to measure, the primary blood volume can be calculated according to the ratio of the blood volume change when the circulation outside the body is performed without water removal, with the following formula.

As it is:

$$\text{blood volume change } (\Delta BV) = BV \text{ (primary blood volume)} \times$$

$$\Delta BV\% \text{ volume inside the circuit}$$

said formula can be change to:

$$\text{primary blood volume } (BV_0) = \text{volume in the circuit}/\Delta BV\%$$

Said primary blood volume corresponds to the sum of the blood volume in the body and the blood volume outside the body.

3. $\Delta BV$

It refers to the BV volume change, and it can be calculated by the following formula:

$$\Delta BV \text{ [BV volume change]} = (\text{Ht at the time of the initiation}$$

$$\text{of dialysis/Ht at the time of measurement}) - 1$$

Said Ht is the abbreviation of Hematocrit showing the bulk ratio of red corpuscle in the whole blood.

4. $\Delta BV\%$

It is the ratio (percentage) of the BV volume change, and as shown in the following formula, the $\Delta BV$ level at the time of measurement is divided by $BV_0$ at the time of the initiation of dialysis and is expressed in percentage.

$$\Delta BV\% = \Delta BV/BV_0 \times 100$$

5. BV%

It is calculated by dividing the BV level at the time of measurement by $BV_0$ at the time of the initiation of dialysis and is expressed in percentage.

$$BV\% = BV \text{ level at the time of measurement}/BV_0 \times 100$$

If the standard blood volume is defined, the target BV% (to maintain) can be calculated from the defined standard blood volume and the blood volume at the beginning of dialysis calculated above. Therefore, the target BV% can be calculated automatically from the primary blood volume.

$$\text{Target BV\%} = \text{standard blood volume/primary blood volume} \times$$

$$100$$

As mentioned above, it is natural that the meaning of BV% level differs by patients. Further, it is possible to define optionally in each establishment, how much time to take to decrease the primary blood volume down to the standard blood volume. For example, as it is shown in Figure 7, by showing the patient's blood volume with the ratio of the blood volume (%BV), the primary blood volume in the graph is determined to be the point A, and the target standard blood volume is determined to be the point B. Therefore, the water removal time to reach the point B from the point A can be defined by monitoring each patient's condition. Depending on the amount of said water removal time, the inclination C of the speed with which the blood volume changes will be either be sharp or mild.

**Industrial Applicability**

According to the present invention, a blood purification apparatus having excellent effects as follows can be obtained.

(1) The blood purification can be carried out accurately by an easy operation.
(2) The blood purification can be carried out safely and physiologically for a living body.
(3) The dialysis efficiency can be improved (elevated), and as a result the extension of the blood purification time can be avoided, the removal amount of medium-molecular-weight waste products is increased.

**Brief Description of Drawings**

Figure 1 is a figure explaining that in the former part of the blood purification process, the purification process is performed by maintaining the blood osmotic pressure continuously in an increasing condition with the use of sodium rich dialysate, and that in the latter part of the purification process the purification process is performed by injecting sodium rich dialysate and normal dialysate into the blood circuit or into the circuit supplying dialysate periodically to form a repeating condition of increasing and decreasing the blood osmotic pressure artificially. Figure 2 (a) is a figure showing a frame format of the appearance of water 1 transferring from cell 2 to blood vessel 3 according to the difference of the osmotic pressure in the blood vessel and in the cells, in the former part of the dialysis. (b) is a figure showing a frame format of the appearance of water 1 transferring from blood vessel 3 to cell 2 according to the difference of the osmotic pressure in the blood vessel and in the cells in the latter part of the dialysis. (c) is a figure showing a frame format of the appearance of water 1 transferring from cell 2 to blood vessel 3 according to the difference of the osmotic pressure in the blood vessel and in the cells in the latter part of the dialysis. Figure 3 is a figure showing a frame format of the transfer of water from the blood vessel into the cell, or in the opposite direction, (a) just before the circulation outside the body, (b) just after the initiation of the circulation outside the body, and (c) after the initiation of the circulation outside the body. In Figure 3, white arrow shows the direction of the transfer of water, and black arrow shows the direction of the transfer of the solute component. Figure 4 is a schematic diagram explaining a method to calculate the patient's primary blood volume (performing water removal). Figure 5 is a schematic diagram explaining another method to calculate the patient's primary blood volume (without performing water removal). Figure 6 is a figure explaining the principle that changes the difference of the osmotic pressure between the patient's blood osmotic pressure and the patient's intracellular osmotic pressure periodically, according to the change or stop of the water removal speed. Figure 7 is a schematic diagram explaining an example to define the speed (inclination) to lower the blood volume from the primary blood volume ($BV_0$) to the standard blood volume (target %BV) in the former part of the dialysis, and in the bottom part of Figure 7, the water removal speed at each blood purification point is shown in the Y axis direction. In Figure 7, A is the primary blood volume (100% BV), B is the standard blood volume (target %BV), C is the speed (inclination) to lower the blood volume from the primary blood volume ($BV_0$) to the standard blood volume (target %BV).

**Best mode of Carrying out the Invention**

Example

The present example is an example of improving the dialysis efficiency by combining sodium infuser and on-line HDF, and is explained according to Figure 1.

In the upper part, the chronological change of the blood indication level is shown, and in the bottom part, the injection condition of sodium rich dialysate is shown. In the blood purification apparatus of the present example, Na infuser and on-line HDF is combined, and also by using UFR controller, the compressing volume from the dialysate and the water removal volume from the dialyzer are made to be the same amount, that is to be balanced. Therefore, the blood osmotic pressure can be changed rapidly, sharply, and also in a wide range. Furthermore, to change the electrolyte of the dialysate circulating in the dialysate circuit, the sodium injector that injects solution comprising rich sodium into the dialysis circuit is suitable.

The sodium injector is disposed on the upper stream side of the hemodialyzer of the dialysate circuit, and is able to circulate solution comprising rich sodium. In the former part of the purification process, the dialysate is made to be in a sodium rich condition to elevate the blood osmotic pressure, and the PRR is thereby increased. Next, in the latter part of the purification process, the sodium injector is controlled so that the dialysate repeates alternatively the sodium rich condition and the standard condition.

In the primary dialysis, by using Na injector, a solution of rich Na is produced. By using said rich Na dialysate, on-line HDF is performed. The blood where the rich Na dialysate is injected becomes hyperosmotic, PRR increases and the water removal speed will be high and it will be possible to elevate the material removal ability from plasma.

Next, when the latter part of the dialysis is started, Na injector is operated intermittently and a rich Na dialysate and a normal dialysate are produced alternatively. By performing on-line HDF with said dialysate, a difference of the osmotic pressure between the cells and the blood is formed artificially. Therefore, water transfer between the cells and the blood can be performed by push & pull, and it becomes possible to exceed the conventional limit of HDF. In the latter part of the dialysis, as a space where the water transfer can be made is formed in both cells and blood vessel, by repeating alternatively an intermittent a rich Na condition and a standard Na condition, an efficient flow in and flow out of water can be made and based on this, the solute that are hard to transfer can be also removed.

When injecting rich Na solution, as the blood would be hyperosmotic than cells, a large amount of water will transfer from cells to plasma. Then, the intracellular osmotic pressure will become hyperosmotic. Next, when it attains the standard concentration of the dialysate, plasma will become an isotonic solution from a hypertonic solution, and the cell side will change to hyperosmotic side. Then, the water transfer from the blood to cells will occur and the intracellular osmotic pressure will decrease. By repeating such operation, and the material removal ability from the cells will be increased at once, and the problem of outflow (removal) time of material from the cells which was a deficit will be solved and the realization of a short time dialysis is approached.

## Claims

1. A blood purification apparatus **characterized** to have a blood osmotic pressure changing means that changes periodically the osmotic gradient between the blood osmotic pressure of a patient and the patient's intracellular osmotic pressure.

2. The blood purification apparatus according to claim 1, wherein the blood purification condition is controlled according to a blood indication level.

3. The blood purification apparatus according to claim 2, wherein the control of the blood purification condition is carried out according to the target control line having blood indication level as an index level.

4. The blood purification apparatus according to either claim 1, 2 or 3, wherein the periodical change of the osmotic gradient between the blood osmotic pressure of a patient and the patient's intracellular osmotic pressure is performed in the latter part of the dialysis operation.

5. The blood purification apparatus according to claim 4, wherein with a blood osmotic pressure changing means, the apparatus has a constitution to carry out purification process by making the blood osmotic pressure continuously in an increasing condition, in the former part of the blood purification process, and to carry out blood purification by forming a repeating condition of increasing and decreasing of the blood osmotic pressure.

6. The blood purification apparatus according to claim 1, 2, 3, 4 or 5, wherein the blood osmotic pressure changing means is a means to form a repeating condition of increasing and decreasing of patient's blood osmotic pressure in the blood circuit, during the blood purification process.

7. The blood purification process according to claim 6, wherein the blood osmotic pressure changing means is a means performed with the change of purification condition in the blood purification process.

8. The blood purification process according to claim 7, wherein the change of the purification condition is a change of water removal speed including the stop of the water removal.

**9.** The blood purification apparatus according to claim 8, wherein the difference of the osmotic pressure between the blood osmotic pressure of a patient and the intracellular osmotic pressure of the patient is changed periodically with the change of the water removal speed.

**10.** The blood purification apparatus according to claim 6, wherein the blood osmotic pressure changing means is performed by injecting directly the osmotic pressure changing material into the blood circuit.

**11.** The blood purification apparatus according to claim 6, wherein the blood osmotic pressure changing means is performed by injecting the osmotic pressure changing material into the dialysate circuit.

**12.** The blood purification apparatus according to claim 1, 2, 3, 4, 5, 6, 7, 8, 9, 10 or 11, wherein the blood purification means is an on-line hemodiafiltration (HDF) that is carried out by balancing the fluid volume flowed in from the dialysate circuit to the blood circuit, and the fluid volume flowed out from the dialysate circuit into the dialysate circuit via a hemodialyzer.

**13.** The blood purification apparatus according to 11 or 12, wherein the blood osmotic pressure changing means is a means of injecting a fluid comprising high content of sodium ion (140-150 mEq/l) and fluid comprising normal sodium ion (140-143 mEq/l) to the dialysate circuit with an sodium injector.

**14.** A blood purification method wherein with a blood osmotic pressure changing means, the purification process is carried out by making continuously the blood osmotic pressure in an increasing condition in the former part of the blood purification process, and the purification process is carried out by forming artificially a repeating condition of increasing and decreasing of the blood osmotic pressure in the latter part of the blood purification process, and said purification process is controlled according to a blood indication level or to a target control line wherein said blood indication level is an index level.

**15.** The blood purification method according to claim 14, wherein the blood osmotic pressure changing means is performed by changing the water removal speed comprising the stop of the water removal or by supplying the osmotic pressure changing material to the blood circuit or to the dialysate circuit.

**16.** A recording medium that has recorded at least the blood purification condition according to the blood osmotic pressure changing means, the blood indication level and the target control line of the blood purification according to the blood indication level.

Figure 1

Former part of the dialysis

Latter part of the dialysis

rich sodium dialysate

rich sodium dialysate

normal dialysate

Figure 2

Former part of the dialysis

Latter part of the dialysis
(repeating)

(a)

(b)

(c)

Figure 3

(a)

(b)

(c)

## Figure 4

reset

$\% \Delta B V_A'$

$\% \Delta B V_B'$

certain water removal (water removal speed B)

certain water removal (water removal speed A)

## Figure 5

stabilization of BV level

n %B V
(blood volume outside the body)

increase of BV level

1 0 0 %B V
(blood volume inside the body)

patient's primary blood volume

Time of the initiation of the dialysis

inflow volume from the cells

Figure 6

Figure 7

| | | |
|---|---|---|
| **INTERNATIONAL SEARCH REPORT** | International application No.<br>PCT/JP02/07777 | |

| A. CLASSIFICATION OF SUBJECT MATTER |
|---|
| Int.Cl⁷ A61M1/34, A61M1/14 |
| According to International Patent Classification (IPC) or to both national classification and IPC |

| B. FIELDS SEARCHED |
|---|
| Minimum documentation searched (classification system followed by classification symbols)<br>Int.Cl⁷ A61M1/34, A61M1/14 |

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2002 |
| Kokai Jitsuyo Shinan Koho | 1971–2002 | Jitsuyo Shinan Toroku Koho | 1996–2002 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | JP 11-221275 A (JMS Co., Ltd.),<br>17 August, 1999 (17.08.99),<br>Full text; Figs. 1 to 9<br>(Family: none) | 1-13 |
| A | WO 00/25804 A2 (The Regents of the University of California),<br>11 May, 2000 (11.05.00),<br>Full text; Figs. 1 to 3<br>& JP 2002-528241 A | 1-13 |
| A | JP 59-115051 A (Toyota Central Research And Development Labo),<br>03 July, 1984 (03.07.84),<br>Full text; Figs. 1 to 8<br>(Family: none) | 1-13 |

| ☒ Further documents are listed in the continuation of Box C. | ☐ See patent family annex. |
|---|---|

| | |
|---|---|
| *  Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>01 November, 2002 (01.11.02) | Date of mailing of the international search report<br>19 November, 2002 (19.11.02) |
|---|---|
| Name and mailing address of the ISA/<br>Japanese Patent Office | Authorized officer |
| Facsimile No. | Telephone No. |

Form PCT/ISA/210 (second sheet) (July 1998)

**EP 1 421 958 A1**

## INTERNATIONAL SEARCH REPORT

International application No.

PCT/JP02/07777

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 57-78866 A (Olympus Optical Co., Ltd.), 17 May, 1982 (17.05.82), Full text; Figs. 1 to 4 (Family: none) | 1-13 |
| A | JP 2001-540 A (JMS Co., Ltd.), 09 January, 2001 (09.01.01), Full text; Figs. 1 to 5 (Family: none) | 16 |
| A | JP 5-115546 A (Asahi Medical Co., Ltd.), 14 May, 1993 (14.05.93), Full text; Figs. 1 to 16 (Family: none) | 16 |

Form PCT/ISA/210 (continuation of second sheet) (July 1998)

17

## INTERNATIONAL SEARCH REPORT

| International application No. |
| --- |
| PCT/JP02/07777 |

**Box I    Observations where certain claims were found unsearchable (Continuation of item 1 of first sheet)**

This international search report has not been established in respect of certain claims under Article 17(2)(a) for the following reasons:

1. ☒ Claims Nos.: 14-15

   *because they relate to subject matter not required to be searched by this Authority, namely:*
   Claims 14 and 15 pertain to methods for treatment of the human body or animal body by surgery or therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:

   *because they relate to parts of the international application that do not comply with the prescribed requirements to such an extent that no meaningful international search can be carried out, specifically:*

3. ☐ Claims Nos.:

   *because they are dependent claims and are not drafted in accordance with the second and third sentences of Rule 6.4(a).*

**Box II    Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest.

☐ No protest accompanied the payment of additional search fees.

Form PCT/ISA/210 (continuation of first sheet (1)) (July 1998)